(19) Europäisches Patentamt
European Patent Office
Office européen des brevets

(11) Numéro de publication: **0 347 276**
**A1**

(12) # DEMANDE DE BREVET EUROPEEN

(21) Numéro de dépôt: **89401514.8**

(22) Date de dépôt: **01.06.89**

(51) Int. Cl.[4]: **C 08 G 75/02**
C 07 D 213/70

(30) Priorité: **14.06.88 FR 8808051**

(43) Date de publication de la demande:
**20.12.89 Bulletin 89/51**

(84) Etats contractants désignés:
**BE CH DE GB IT LI LU NL SE**

(71) Demandeur: **CENTRE D'ETUDE DES MATERIAUX ORGANIQUES POUR TECHNOLOGIES AVANCEES**
**Autoroute Lyon-Vienne Echangeur de Solaize Vernaison**
**Solaize**
**F-69390 Vernaison (FR)**

(72) Inventeur: **Romdhane, Hatem Ben**
**21, rue Salaheddime**
**El Ayoubi 2080 Ariana (TN)**

**Boileau, Sylvie**
**38, rue des Cordelières**
**F-75013 Paris (FR)**

**Bartholin, Michel**
**33, chemin des Rossignols**
**F-69390 Vernaison (FR)**

**Sillion, Bernard**
**93, rue Juliot Curie**
**F-69005 Lyon (FR)**

(74) Mandataire: **Andreeff, François**
**INSTITUT FRANCAIS DU PETROLE 4, avenue de Bois-Préau**
**F-92502 Rueil-Malmaison (FR)**

(54) **Compositions d'oligomères polythiopyridines à terminaisons halogénées, leur préparation et leur utilisation pour la fabrication de résines thermostables.**

(57) Compositions d'oligomères de type polythiopyridine à terminaisons halogénées comprenant au moins un oligomère représenté par la formule générale :

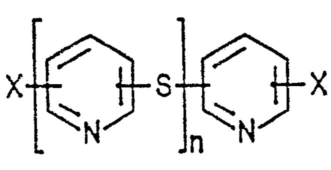

dans laquelle les valences des cycles pyridiniques sont en positions 2,4, 2,6 ou 3,5, n est un nombre entier de 2 à 20 et X est un atome d'halogène. Elles présentent en général une masse moléculaire moyenne en nombre de 200 à 10 000.

Elles peuvent être préparées par réaction de sulfure de sodium avec une dihalogénopyridine, par exemple dans un rapport molaire d'environ 1,05 à 1,5.

Elles peuvent être utilisées pour la fabrication de résines thermostables.

EP 0 347 276 A1

## Description

# COMPOSITIONS D'OLIGOMERES POLYTHIOPYRIDINES A TERMINAISONS HALOGENEES, LEUR PREPARATION ET LEUR UTILISATION POUR LA FABRICATION DE RESINES THERMOSTABLES.

La présente invention a pour objet de nouvelles compositions d'oligomères polythiopyridines terminés par des fonctions halogènes à chaque extrémité de la chaîne. Elle concerne également un procédé de préparation de ces oligomères basé sur une substitution de dihalogéno-pyridine par les sulfures alcalins, catalysée par des catalyseurs de transfert de phase.

Les oligomères α, ω-difonctionnels constituent une classe de produits de plus en plus demandés qui trouvent des applications dans la préparation des polymères de spécialités. Par exemple lorsque les fonctions portées par les extrémités de chaînes peuvent être engagées dans des réactions de condensation (fonction amine, alcool, acide, phénol...etc), les oligomères peuvent être utilisés pour la préparation de polycondensats séquencés élastomères ou thermoplastiques. Lorsque les fonctions portées par les extrémités de chaînes sont des fonctions insaturées réagissant par des réactions d'addition, les oligomères correspondants donnent naissance par réactions radicalaire catalysée ou thermique à des réseaux réticulés utilisables comme colles ou matrices de composites.

Le développement des matériaux organiques stables à température élevée est lié aux nouveaux besoins des industries de pointe comme les industries électroniques ou les industries de l'espace. Des résines fusibles réagissant par voie thermique pour conduire à des réseaux réticulés, utilisables en service continu à une température supérieure à 200°C sont actuellement recherchées par ces industries. Les fonctions réactives terminales utilisées sont soit des doubles liaisons de type maléique ou nadique (endométhylène tétrahydrophtalique) soit des insaturations acétyléniques. Les oligomères sur lesquels sont fixées ces fonctions terminales peuvent être des oligomères de type éthersulfone, ou de type hétérocyclique comme les imides ou les quinoxalines.

Les caractéristiques de mise en oeuvre de ces résines et les propriétés mécaniques et thermiques des réseaux réticulés qui en dérivent dépendent dans une très large mesure de la nature chimique de l'oligomère qui porte les fonctions réactives. C'est en effet l'oligomère qui va déterminer d'une part la température de fusion et d'autre part la température de transition vitreuse de la chaine entre noeuds après réticulation.

Par exemple les résines éthersulfones acétyléniques fondent en général à une température inférieure à 120°C mais la température de transition vitreuse du réseau est limitée à 200°C. Par contre les oligomères imides fondent à 190°C et la transition vitreuse du réseau correspondant est supérieure à 350°C.

De nombreuses recherches ont pour objet d'obtenir des oligomères α,ω-difonctionnels précurseurs de résines thermodurcissables.

On attend de ces résines une température de fusion basse (inférieure à 130°C), une bonne réactivité entre 150 et 200°C de manière à disposer d'une "fenêtre de mise en oeuvre" large et une stabilité dimensionnelle du réseau réticulé supérieure à 300°C.

L'objet de la présente invention est de décrire des oligomères α,ω-dihalogénés précurseurs de résines thermostables. Ces oligomères sont caractérisés par des enchaînements sulfure de pyridylène.

Le polysulfure de phénylène est un produit industriel obtenu par substitution nucléophile de dihalogéno-benzène par le sulfure de sodium. Cette réaction conduite à haute température en solvant aprotique fournit un polymère cristallin dont les caractéristiques et les possibilités d'application sont désormais bien connues. ("Les polymères organiques utilisables à température élevée" Ed. Technip, 1983, p. 51).

La préparation de nouveaux oligomères de thiop.phénylène- dicarboxytéléchélique a été décrite dans le brevet français FR-B-2470780. Ces produits ont été utilisés pour la préparation de copolyesters et de copolyamides dont ils améliorent la résistance aux solvants et la stabilité thermique.

Des oligomères contenant les enchaînements pyridylène ont été préparés par réaction de dihalogénopyridine avec différents nucléophiles.

Dans une demande de brevet antérieure, l'organisme demandeur a décrit des oligomères obtenus par réaction de dichloro ou dibromo-2,6 pyridine sur des bisphénols. Ces oligoéthers de pyridylène-2,6 α,ω-dihalogénés sont transformés en dérivés acétyléniques.

En ce qui concerne les polymères ou oligomères contenant l'enchaînement sulfure de pyridylène, Tsuda et coll. (Journal of Polym. Sci., Polym. Letters, Ed. 21, 847 (1983)) ont décrit la réactivité des bisthiols aliphatiques sur les dichloro et dibromo-2,6 pyridine en précisant l'absence de réactivité des bisthiols aromatiques.

L'augmentation de la réactivité de nucléophile au moyen de la catalyse par transfert de phase a fait l'objet de nombreux travaux (Paul Caubère, Le transfert de phase et son utilisation en chimie organique, Masson (1982)). Cette technique a été appliquée à la réaction du sulfure de sodium sur des dérivés dihalogénés en vue de l'obtention de polysulfure aliphatique (G.R. Petit J. Polym. Sci. ; Polym. Chem. Ed., 18, 345, 1980 ; M. Ueda et Coll. Macromolecules, 15, 248, 1982). Plus récemment, il a été montré que la catalyse par transfert de phase permettait la réaction du trichlorométhylbenzène avec le sulfure de sodium (L.H. Tagle et Coll., J. Polym. Sci., Polym. Chem. Ed.,24, 495, 1986).

La plus faible réactivité des halogénures aromatiques vis-à-vis de la substitution nucléophile pouvait laisser croire que la catalyse par transfert serait inopérante et de fait on ne relève aucune indication de ce type de réaction. Au contraire, la réaction du chloro-2 nitro-5 pyridine sur le sulfure de sodium

conduisant au bis-(nitro-5 pyridyl-2 sulfure) met en évidence la nécessité d'activer l'halogène par une groupe très attracteur (J. Metzger et Coll., C.R. Acad. Sci. Paris, Ser. C, 289, 203, 1979).

Les compositions d'oligomères de la présente invention peuvent être définies comme comprenant au moins un oligomère répondant à la formule générale

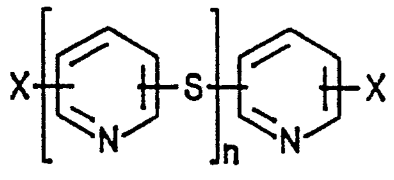

dans laquelle les valences des cycles pyridiniques sont en position 2, 4, ou 2,6, ou 3,5 ; n est un nombre entier habituellement de 2 à 20 et de préférence de 8 à 15. X est un halogène, de préférence le chlore ou le brome.

Leur masse moléculaire moyenne en nombre peut aller de 200 à 10 000. Les compositions d'oligomères de l'invention peuvent être obtenues par réaction d'une dihalogénopyridine avec du sulfure de sodium, de préférence en milieu anhydre dans un rapport en général supérieur à 1, habituellement d'environ 1,05 à 1,5 et de préférence de 1,07 à 1,125.

Parmi les dihalogénopyridines utilisables dans la préparation des compositions de l'invention, on citera la dichloro-2,6 pyridine, la dibromo-2,6 pyridine, les mélanges de dichloro-2,4 et 2,6 pyridines et les mélanges de dibromo-2,4 et 2,6 pyridines, la dichloro-3,5 pyridine et la dibromo-3,5 pyridine, ainsi que les mélanges de dihalogéno (dibromo et dichloro)-2,6, 2,4 et 3,5 pyridines.

Le sulfure de sodium disponible habituellement est le produit hydraté contenant neuf molécules d'eau de cristallisation. Ce produit, pour être utilisé dans la préparation des compositions de l'invention, doit être déshydraté par exemple par un traitement thermique à une température de 100 à 250° C et sous un vide de $10^{-3}$ à $10^{-5}$ mm de mercure (de 133 à 1,33mPa).

La réaction est avantageusement catalysée par des agents de transfert de phase, en particulier ceux qui sont efficaces pour la catalyse solide-liquide ; parmi ceux-ci, on pourra employer les agents complexants sélectifs des cations alcalins, en particulier les éther couronnes et les cryptants. Ces catalyseurs ont un effet particulièrement sensible lorsque la réaction de condensation est opérée en absence de solvant. Le catalyseur est employé habituellement à une concentration molaire allant de 2 à 8 % par rapport à la dihalogénopyridine.

On peut également réaliser la préparation des oligomères de l'invention au sein d'un solvant organique polaire. On utilisera de préférence le diméthylsulfoxyde et le diméthylformamide ; les réactifs sont alors utilisés à des concentrations élevées, allant par exemple de 5 à 10 moles par litre de solvant. La réaction est habituellement conduite à une température allant d'environ 100 à 150° C.

Après réaction, les produits peuvent être isolés par les méthodes habituelles comprenant par exemple un lavage à l'eau pour éliminer les sous-produits tels que les halogénures de sodium qui se forment dans la réaction. Les produits peuvent être caractérisés par détermination des masses moléculaires moyennes en nombre Mn et en poids Mw et par analyse élémentaire. Les composés de structure générale (I) sont des intermédiaires qui peuvent être transformés par substitution des halogènes terminaux.

Les exemples suivants illustrent l'invention sans en limiter la portée.

Exemple 1 : Préparation d'un oligomère α,ω -dibromé

On déshydrate du sulfure de sodium contenant 9 molécules d'eau par un traitement thermique à 180° C sous un vide de $10^{-3}$ mm de mercure (133 mPa) pendant 48 h.

La pureté du sulfure de sodium ainsi séché est de 90 % (détermination par dosage iodométrique).

Un mélange de dibromo-2,6 pyridine (17 mmoles), 1,62 g de sulfure de sodium (18,7 mmoles) et 0,625 g (1,7 mmole) de dicyclohexyl-18 couronne-6, sont chauffés pendant 4 h à 120° C. Après retour à température ambiante, l'oligomère est extrait au chloroforme et la solution chloroformée abondamment lavée à l'eau.

Après évaporation, on recueille l'oligomère avec un rendement de 50 %. Cet oligomère a les caractéristiques suivantes : Mn = 900, Mw = 1500 et possède un atome de brome à chaque extrémité de la chaîne macromoléculaire.

Exemple 2 : Effet de l'absence de catalyseur de transfert de phase sur la condensation à l'état solide.

Avec les réactifs et dans les conditions de l'exemple 1 on effectue la réaction sans le dicyclohexyl-18 couronne-6.

Après traitement, on n'isole qu'une très faible quantité de dimère et on recueille la quasi-totalité de la dibromopyridine introduite dans le milieu réactionnel.

Exemple 3 :Préparation d'un oligomère α, ω -dibromé dans le diméthyl-sulfoxyde (DMSO).

On utilise le sulfure de sodium et la dibromopyridine décrite dans l'exemple 1. 10 g de dibromo-2,6 pyridine (42,2 mmole), 3,657 g de sulfure de sodium (42,2 mmole), 1,56 g de dicyclohexyl-18 couronne-6 et 8 ml de diméthylsulfoxyde sont chauffés en atmosphère inerte à 120° C pendant 6 h avec une agitation à 600 tours par minute.

Après un traitement tel que décrit dans l'exemple 1, on recueille 4,047 g (80 %) d'un oligomère dont les caractéristiques sont les suivantes : Mn = 1400 ; Mw = 1900. Avec un atome de brome à chaque extrémité de la chaîne.

Exemple 4 : Effet de la concentration dans le diméthylsulfoxyde.

Dans les conditions de l'exemple 3, à l'exception de la quantité de DMSO qui est seulement de 4 ml, on obtient 4,864 g de l'oligomère dont les caractéristiques sont les suivantes : Mn = 1300 ; Mw = 1700.

Exemple 5 : Sélectivité du catalyseur.

Dans les conditions de l'exemple 3, on remplace le dicyclohexyl-18 couronne-6 par le dicyclohexyl-15 couronne-5.

Après traitement, on ne recueille que 1,242 g, (54 %) d'un oligomère α,ω-dibromé de masse Mn = 1300 et Mw = 1700.

Exemple 6 :Préparation d'un oligomère α,ω -dibromé dans le diméthylformamide (DMF).

Dans les conditions décrites dans l'exemple 3, mais en remplaçant les 8 ml de DMSO par 8 ml de DMF, on recueille 4,120 g (81 %) d'un oligomère caractérisé par Mn = 1400 et Mw = 1800.

## Revendications

**1/** Composition d'oligomères de type polythiopyridine à terminaisons halogénées caractérisée en ce qu'elle comprend au moins un oligomère répondant à la formule générale :

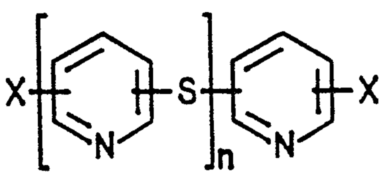

dans laquelle les valences des cycles pyridiniques sont en positions 2,4, 2,6 ou 3,5 ; n est un nombre entier de 2 à 20 et X est un atome d'halogène. **2/** Composition d'oligomères selon la revendication 1, caractérisée en ce que dans ladite formule (I) n est un nombre entier de 8 à 15 et X représente un astome de chlore ou de brome. **3/** Composition d'oligomères selon l'une des revendications 1 et 2, caractérisée en ce qu'elle présente une masse moléculaire moyenne en nombre de 200 à 10 000. **4/** Procédé de préparation d'une composition d'oligomères selon l'une des revendications 1 à 3, caractérisé en ce qu'il comprend la réaction de sulfure de sodium avec un excès de dihalogénopyridine. **5/** Procédé selon la revendication 4, caractérisé en ce que la proportion molaire de la dihalogénopyridine au sulfure de sodium est d'environ 1,05 à 1,5. **6/** Procédé selon l'une des revendications 4 et 5 caractérisé en ce que la réaction est effectuée en présence d'un agent de transfert de phase. **7/** Procédé selon l'une des revendications 4 à 6, caractérisé en ce que la réaction est effectuée au sein d'un solvant organique polaire. **8/** Procédé selon l'une des revendications 4 à 7, caractérisé en ce que la réaction est effectuée à une température d'environ 100 à 150° C.

Office européen des brevets

# RAPPORT DE RECHERCHE EUROPEENNE

Numero de la demande

EP 89 40 1514

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A,D | FR-A-2 470 780 (INSTITUT FRANCAIS DU PETROLE)<br>* revendication 1 * | 1 | C 08 G 75/02<br>C 07 D 213/70 |
| A,D | JOURNAL OF POLYMER SCIENCE, POLYMER LETTERS EDITION vol. 21, no. 10, octobre 1983, pages 847-851; S. KONDO et al.: "Nucleophilic Substitution Polymerization of Pyridines: Condensation Polymerization of Dihalopyridines with Dithiols" | 1 | |
| A | DE-A-2 016 630 (TH. GOLDSCHMIDT AG)<br>* revendication 1 * | 1 | |
| A | FR-A-1 326 625 (IMPERIAL CHEMICAL INDUSTRIES LTD.)<br>* résumé * | 1 | |
| A | PATENT ABSTRACTS OF JAPAN vol. 2, no. 72 (C-78), 31 mai 1978; & JP - A - 53 27695 (HITACHI SEISAKUSHO K.K.) 15.03.1978 | 1 | **DOMAINES TECHNIQUES RECHERCHES (Int. Cl.4)** |
| A,D | JOURNAL OF POLYMER SCIENCE, POLYMER CHEMISTRY EDITION vol. 24, 1986, pages 495-501; L. H. TAGLE et al.: "Polymerization by Phase-Transfer Catalysis 3: Poly(1,4-Oxylylene Thioethers) Synthesis"<br>-/- | 4,6 | C 07 D 213/00<br>C 08 G 75/00 |

Le présent rapport a été établi pour toutes les revendications

| Lieu de la recherche | Date d'achèvement de la recherche | Examinateur |
|---|---|---|
| BERLIN | 11-09-1989 | HASS C V F |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 89 40 1514

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A,D | MACROMOLECULES vol. 15, no. 2, 1982, pages 248-251; M. UEDA et al.: "Synthesis of Poly(aliphatic sulfides) by Polycondensation of Sodium Sulfide with Dibromoalkanes in the Presence of Quaternary Onium Salts" --- | 4,6 | |
| A,D | TABLES DES COMPTES RENDUS DES SEANCES DE L'ACADEMIE DES SCIENCES Ser. C, vol. 289, 1979, pages 203-206; H. ALSAIDI et al.: "Catalyse par transfert de phase en chimie heterocyclique. Substitutions de chloronitropyridines par des carbanions et des nucleophiles soufres et oxygenes" ----- | 6 | |

DOMAINES TECHNIQUES RECHERCHES (Int. Cl.4)

Le présent rapport a été établi pour toutes les revendications

| Lieu de la recherche | Date d'achèvement de la recherche | Examinateur |
|---|---|---|
| BERLIN | 11-09-1989 | HASS C V F |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant